# EUROPEAN PATENT SPECIFICATION

(11) **EP 1 693 383 B1**
(45) Date of publication and mention of the grant of the patent: **09.10.2013**
(21) Application number: 05016576.0
(22) Date of filing: 29.07.2005
(51) Int. Cl.: G01N 33/574, C07K 16/00, C07K 16/30, C07K 16/40

(54) **Antibodies for use in identifying prostate cancer**
Antikörper zur Erkennung von Prostatakrebs
Anticorps pour l'identification du cancer de la prostate

(30) Priority: 18.02.2005 EP 05003596; 13.07.2005 EP 05015253
(43) Date of publication of application: 23.08.2006
(73) Proprietor: Universitätsklinikum Freiburg, 79106 Freiburg (DE)
(72) Inventor: Schüle, Roland, Prof., 79367 Weisweil (DE); Metzger, Eric, Dr., 68600 Neuf-Brisach (FR); Buettner, Reinhard, Prof., 53127 Bonn (DE)
(74) Representative: Maiwald Patentanwalts GmbH

(56) References cited:
- SHI YUJIANG ET AL: "Histone demethylation mediated by the nuclear amine oxidase homolog LSD1" CELL, CELL PRESS, CAMBRIDGE, NA, US, vol. 119, no. 7, 29 December 2004 (2004-12-29), pages 941-953, XP002321632 ISSN: 0092-8674

## Description

The present invention relates to a method for identifying and/or scoring prostate carcinomas and the use of anti-LSD1 antibodies for identifying and/or scoring prostate cancer in a mammal.

The androgen receptor (AR) is a member of the steroid hormone receptor family of transcription factors which regulate diverse biological functions including cell growth and differentiation, development, homeostasis and various organ functions in a mammal, particularly in a human. By binding suitable ligands like androgens to the ligand binding domain, functions of the AR are activated which are essential for the differentiation, development and maintenance of male or female reproductive organs and non-reproductive organs (as, for example, the prostate or the mammae).

Transcriptional regulation by nuclear receptors such as the androgen receptor (AR) involves interaction with multiple factors that act in both a sequential and combinatorial manner to reorganize chromatin⁵. Central to this dynamic reorganization is the modification of core histones. The N-terminal tails of histones are subject to various covalent modifications such as acetylation, phosphorylation, ubiquitination and methylation by specific chromatin-modifying enzymes⁶. Histone methylation at specific lysine residues is linked to both transcriptional repression and activation⁶. When searching for new AR interacting proteins, Lysine specific demethylase 1 (LSD1)⁷ was found to be one example of the chromatin-modifying enzymes.

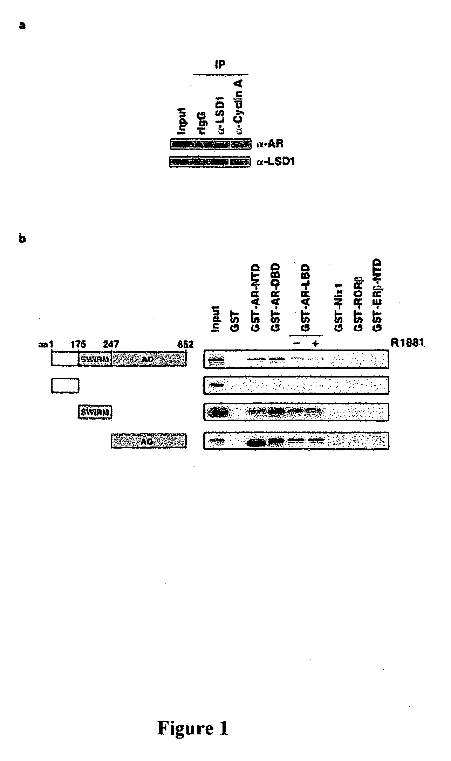

LSD1 contains a centrally located swirm domain which functions as a putative protein-protein interaction motif, and also contains a C-terminal amine oxidase (AO) domain that harbours the demethylase activity⁷ (Fig. 1b). Endogenous LSD1 and AR associate *in vivo* in androgen-sensitive tissues such as testis (Fig. 1a). To map the interaction domain between LSD1 and AR *in vitro,* GST pull-down analyses with labelled LSD1 and mutants thereof together with GST-AR fusion proteins were performed. As shown in figure 1b, full-length LSD1, as well as the swirm domain (LSD1 175-246) and the AO domain (LSD1 247-852) associate with either the N-terminus (NTD), the DNA binding domain (DBD), or the ligand-binding domain (LBD) of AR. In contrast, neither the N-terminus of LSD1 (LSD1 1-174) nor the GST control interact with AR.

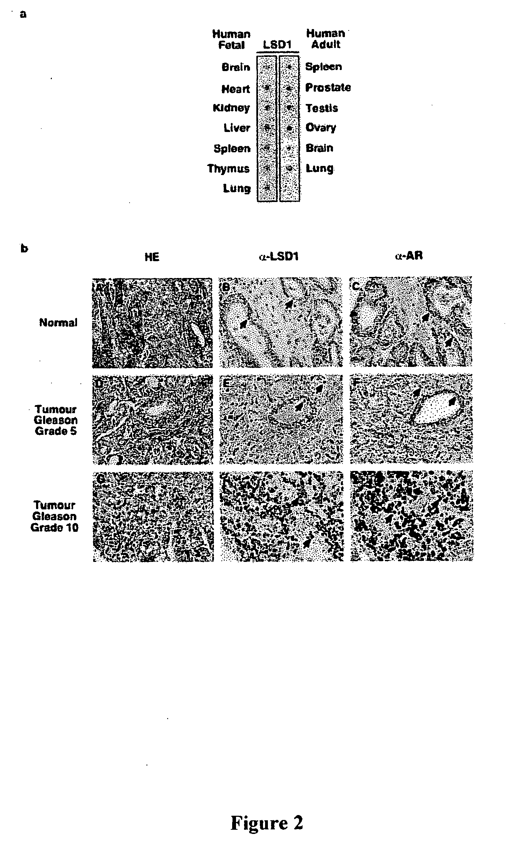

It was now surprisingly found that the demethylating enzyme LSD1 is expressed ubiquitously in human and murine fetal and adult tissues (Figure 2a and data not shown). Furthermore, it was also detected that LSD1 is found in the same cells (and in the same sub-cellular areas) where the AR is located (Figures 2c, d). In the course of the research resulting into the present invention, the above (and further) findings led to the conclusion that the demethylating enzyme LDS1 may exert a controlling influence on androgen-dependent gene expression. Furthermore, it was found that specific LSD1 antibodies may be used to control demethylase activity and thereby regulate the AR. Thus, a specific modulation of LSD1 activity might by a promising therapeutic target.

Prostate cancer represents the most frequent malignant disease in men worldwide and the second leading cause of death from malignant tumors³. The incidence is strongly related to age: While being very rare below the age of 50 years the incidence rises to approximately 1150 cases per 100,000 males at the age of 80⁴. In parallel there is a significant increase in overall incidence. In the year 2000, there were 92,000 new cases and it is estimated that this figure will increase to 120,000 in the year 2020³.

A peculiarity of prostate cancer is a relatively high portion of latent cancers that will not progress to clinically manifested disease and therefore require no therapy¹. These tumors also show an age-dependent increase in incidence from 10% at the age of 50 to 60% in patients older than 80 years. It is estimated that only 1/3 to 1/5 of all prostate cancers progress to clinically relevant disease. These data highlight the clinical need to distinguish reliably between progressive and non-progressive carcinomas.

The clinical outcome of prostate cancer is strongly related to its differentiation and malignancy grade. In particular the Gleason scoring system, presently the most common prostate cancer scoring system, makes use of the increasingly disturbed normal tissue architecture in high grade carcinomas. However, the Gleason scoring system reveals significant inter-observer variation and is difficult to assess reliably in small biopsies. Although a large number of tumor-suppressor genes and oncogenes have been identified and analysed in prostate cancers, the molecular mechanisms, which lead to dissolution of glandular structures and invasion, are largely unknown. Thus, there is a clinical need for molecular factors discriminating between progressive and non-progressive carcinomas in biopsies.

In the article entitled "Histone demethylation mediated by the nuclear amine oxidase homolog LSD1" (Cell, vol. 119, pages 941-953) the authors Yujiang Shi *et al.* provide evidence that LSD1 functions as a transcriptional corepressor. When LSD1 expression was inhibited by RNAi, an increase in histone H3 lysine 4 methylation and a derepression of target genes could be observed. Therefore, the authors suggest LSD1 represses transcription via histone demethylation.

We have recently observed that the androgen receptor-interacting protein LSD1 is overexpressed in dedifferentiated and progressive prostate carcinomas. Therefore, we analysed the expression of LSD1 in a cohort of 99 patients with prostate cancers of different biology. Group A patients had tumours with clinical stages of T2c or less and Gleason scores of 6 or less (low risk cancers). Group B patients had tumours with any clinical stage and Gleason score 8, 9, or 10 (high risk cancers). 29 different antigens were stained by immunohistochemistry in these cancers and quantified according to the number of positive cancer cells and the staining intensity on a numerical scale from 0 to 300. These studies identified LSD1 as the most discriminatory antigen (p<0.001) that was strongly overexpressed in carcinomas of the high risk group.

From these data we claim that immunohistochemical staining of LSD1 in prostate carcinomas is the most discriminatory surrogate marker to identify high risk carcinomas at risk for systemic progression.

The invention relates to a method as defined in claim 1, namely to a method for identifying and/or scoring prostate carcinomas, said method comprising the step of immunostaining tissues, cells, body fluids and/or protein extracts relating to prostate cancer including employing anti-LSD1 antibodies, and quantifying the LSD1 amount in said tissues, cells, body fluids and/or protein extracts.

The invention also relates to the use of at least one antibody for identifying and/or scoring prostate carcinomas in a mammal as defined in claim 6.

The invention is further explained in the following description by referring to the Figures. In the Figures,
Figure 1 shows the following: LSD1 interacts with AR *in vivo* and *in vitro.* **a**, AR co-immunoprecipitates with LSD1. Extracts from mouse testis were immunoprecipitated with α-LSD1 or α-cyclin A antibodies and rabbit IgG as control. Western blots were decorated with α-AR and α-LSD1 antibodies. **b**, GST pull-down assays were performed with labelled LSD1 mutants and the corresponding bacterially expressed GST-AR fusion proteins. GST, GST-Nix1, GST-ROR□, and GST ERβ-NTD proteins were used as control. (NTD; N-terminal domain, DBD; DNA-binding domain, LBD; ligand-binding domain).
Figure 2 shows LSD1 expression analyses: **a**, Expression of LSD1 mRNA in human tissues was examined by Northern blot analyses on a Human Multiple Tissue Expression Array. **b**, lmmunohistochemical staining of LSD1 and AR in human normal and tumour prostate. LSD1 (B, E, H) and AR (C, F, I) immunoreactivity is detected in the secretory epithelium of normal prostate (B, C, arrows) and tumour cells (E, F, H, I, arrows). Hematoxilin-eosin (HE) stained sections are shown (A, D, G). All sections were taken from the same radical prostatectomy specimen. Magnification: x250.

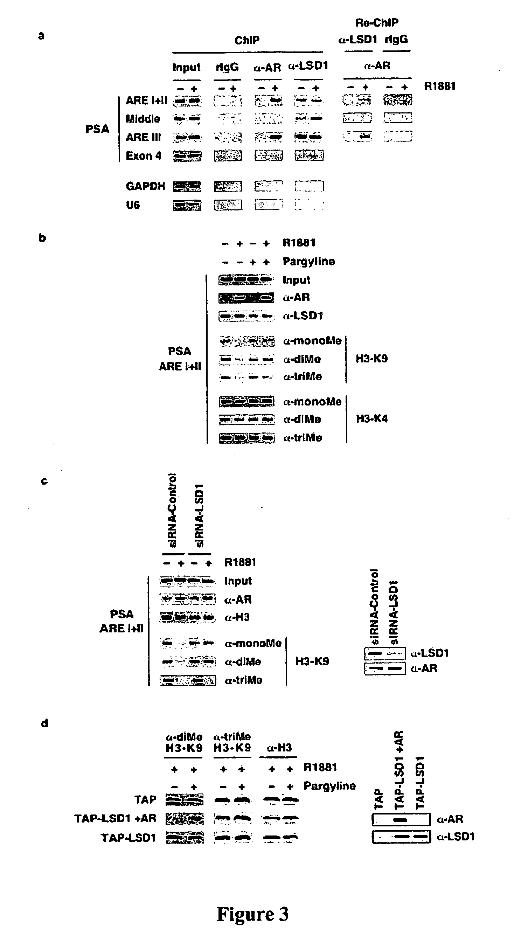

Figure 3 shows how LSD1 interacts with chromatin. LNCaP cells were incubated with or without R1881 (**a, b, c**), treated with or without pargyline (**b**), or transfected with siRNA (**c**). ChIP or Re-ChIP was performed with the indicated antibodies. The precipitated chromatin was amplified by PCR using primers flanking the promoter region (ARE I+II), the middle region (middle), the enhancer region (ARE III), exon 4 of the *PSA* gene, or the promoters of the *GAPDH* and *U6* genes. siRNA-mediated knockdown of LSD1 is verified by Western blot analysis (**c**, right panel) using α-AR and α-LSD1 antibodies. **d**, Native nucleosomes from HeLa cells were incubated in the presence of R1881 with either purified TAP, TAP-LSD1/AR, or TAP-LSD1 complexes with or without pargyline. Western blots were decorated with the indicated antibodies (left panel). The presence of LSD1 and AR in the TAP purified protein complexes was verified by Western blotting using αAR and α-LSD1 antibodies (right panel).

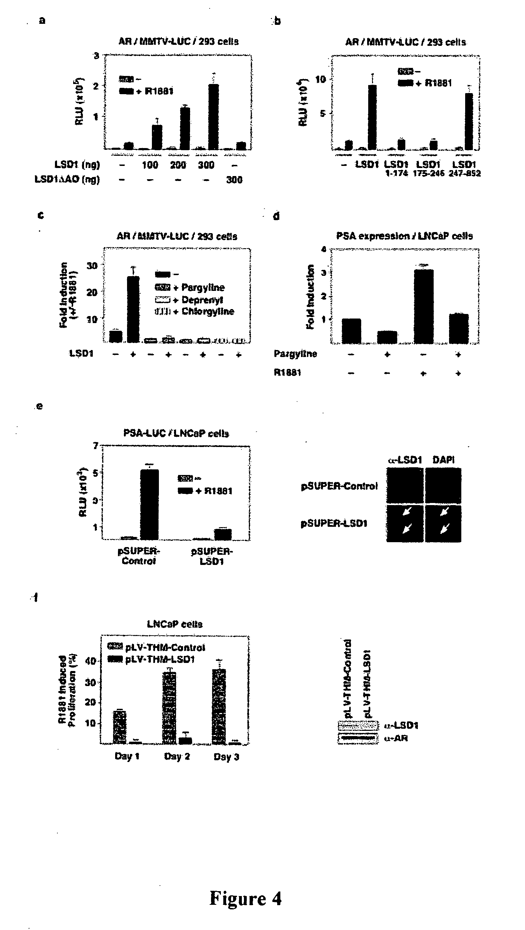

Figure 4 shows how LSD1 controls AR-induced transcriptional activity and cell proliferation. 293 **(a, b, c),** or LNCaP **(e)** cells were transfected with the indicated AR-dependent reporters in presence of AR expression plasmid (**a-c**). Cells were treated with or without R1881, pargyline, deprenyl, or clorgyline. LSD1-induced ligand-dependent activation of AR (**a**) is mediated by the AO domain (LSD1 247-852, **b**) and blocked by monoamine oxidase inhibitors (**c**). Pargyline also reduces endogenous *PSA* gene expression in LNCaP cells as quantified by qRT-PCR **(d).** In LNCaP cells, siRNA-mediated LSD1 knockdown reduces AR activity (**e**, left panel). LSD1 knockdown inhibits R1881-induced LNCaP cell proliferation (**f**, left panel). Knockdown of LSD1 is verified by immunofluorescence (**e**, right panel, arrows) and Western blot analysis (**f**, right panel) using α-AR and α-LSD1 antibodies. Bars represent mean +SD (n>5).

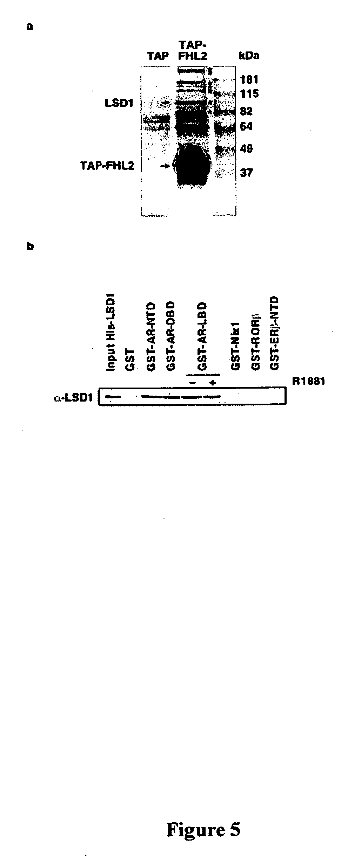

Figure 5 shows: **a,** Coomassie blue staining reveals that LSD1 (arrow) is copurified with TAP-FHL2 (arrow) during tandem affinity purification. Asterisks represent proteins that specifically co-purify with TAP-FHL2 but not with the TAP control. **b**, Western blot analysis using α-LSD1 antibody show that bacterially expressed and purified His-LSD1 interacts with bacterially expressed and purified GST-AR fusion proteins but not with the GST, GST-Nix1, GST-RORβ, and GST ERβ-NTD control proteins. (NTD; N-terminal domain, DBD; DNA-binding domain, LBD; ligand-binding domain).

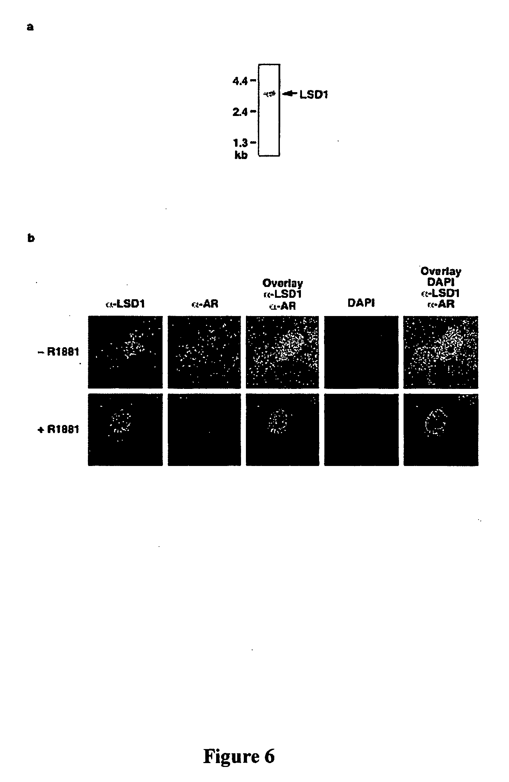

Figure 6 shows LSD1 expression analyses. **a**, Expression of LSD1 in human tissues was examined by Northern blot analyses of human testis mRNA. **b**, Confocal laser scanning images shows sub-cellular localisation of endogenous LSD1 and AR in human LNCaP prostate tumour cells. AR (red) co-localises with LSD1 (green) in the nucleus upon addition of the AR agonist R1881.

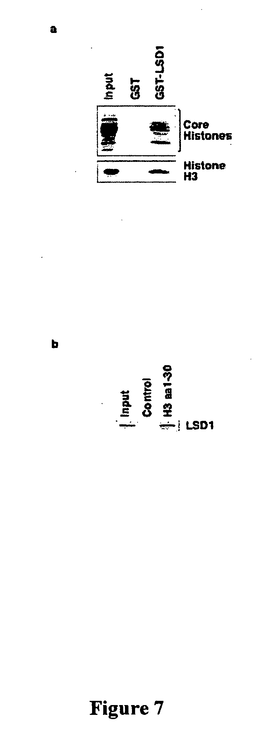

Figure 7 shows that LSD1 interacts with chromatin. **a**, Coomassie blue staining reveals interaction of bacterially expressed GST-LSD1 with core histones and histone H3 *in vitro. **b**, In vitro* translated ³⁵S-methionine labeled LSD1 interacts with sepharose coupled N-terminal tail of histone H3.

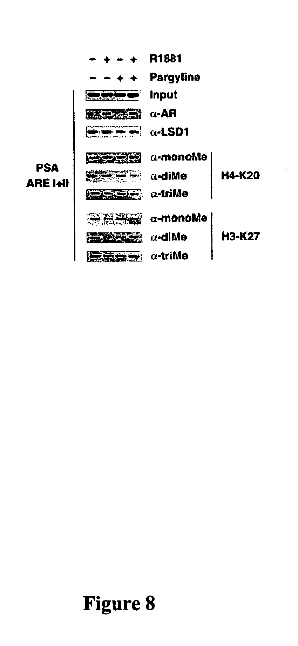

Figure 8 LNCaP cells were incubated with or without R1881 and treated with or without pargyline. ChIP assays were performed with the indicated antibodies. The precipitated chromatin was amplified by PCR using primers flanking the promoter region (ARE I+II) of the *PSA* gene.

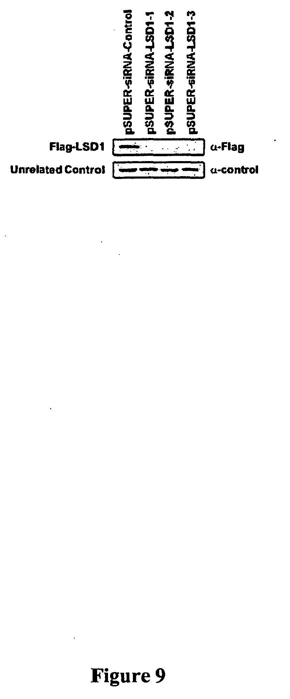

Figure 9 shows siRNA mediated knockdown of LSD1. 293 cells were cotransfected with Flag-LSD1 and pSUPER constructs expressing siRNA against LSD1. The different DNA target sequences for siRNA are: LSD1-1 5'-CGGACAAGCTGTTCCTAAA-3'; LSD1-2 5'-GAACTCCATCAGCAATACA-3'; LSD1-3 5'-CACAAGGAAAGCTAGAAGA-3'; unrelated control 5'-CTTGCTATGAGAACAAATT-3'. 24 hours after transfection, cells were harvested and cell lysate was analysed in Western blot for expression of Flag-LSD1. The Western blot was decorated with an α-Flag antibody. The siRNA corresponding to LSD1-3 was used for the experiments in figure 3 and 4.

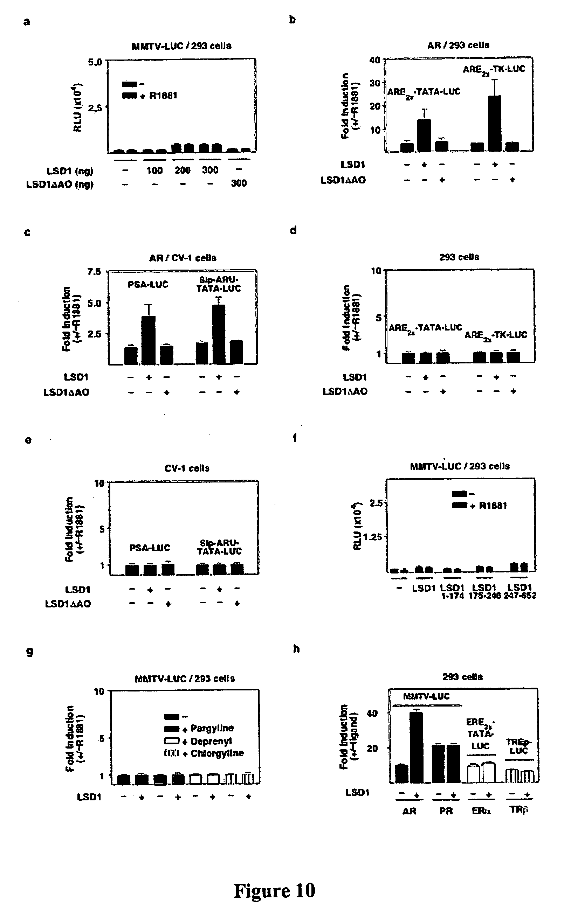

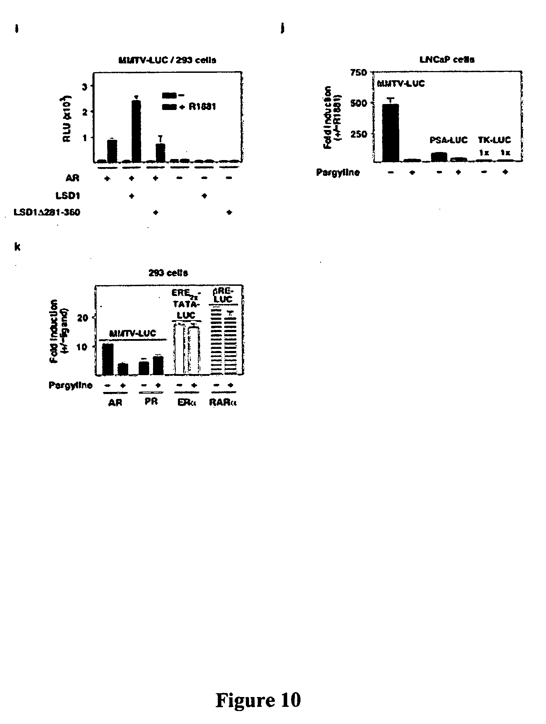

Figure 10 shows the specificity of LSD1 in the control of AR-induced transcriptional activity. 293 (a, b, d, f, g, h, i, k), CV-1 (c, e), and LNCaP (j) cells were transfected with the indicated reporters in the absence of AR (a, d, e, f, g) or in presence of AR, PR, αRα, RARα, or TRα expression plasmids (b, c, h, i, k). Cells were treated with or without R1881, R5020, E₂, T3, all*-trans* RA, pargyline, deprenyl, and clorgyline. LSD1 induces ligand-dependent activation of AR reporters only in presence of AR (b, c) but not in absence of AR (a, d, e, f, g). LSD1 does not influence activation of other nuclear hormone receptors (h). The AO deletion mutant LSD1Δ281-360 fails to activate AR-dependent gene expression (i). Pargyline blocks AR-dependent reporter genes (j) but fails to block other nuclear hormone receptors (k). Bars represent mean +SD (n>5).

The following detailed description of the invention refers to the invention in its broadest sense, but also to specific embodiments which may be preferred due to the excellent and surprising results achieved.

The surprising finding on which the present invention is based is the fact that LSD1 is overexpressed in dedifferentiated and progressive prostate carcinomas, while it is not in prostate cells of less progressive stage carcinomas. When immunohistochemically staining prostate carcinoma tissue, prostate carcinoma cells, body fluids of (or containing) prostate carcinoma tissue and/or cells and/or protein extracts thereof, the staining intensity is a representative measure for the amount of LSD1 expressed which, in turn, is a good measure for the stage of carcinoma progression. In accordance with such a finding, anti-LSD1 antibodies are preferred for the practice of the invention.

The invention relates to a method for identifying and/or scoring prostate carcinomas, said method comprising the step of immunostaining tissues, cells, body fluids and/or protein extracts relating to prostate cancer including employing anti-LSD1 antibodies and quantifying the LSD1 amount in said tissues, cells, body fluids and/or protein extracts.

As was surprisingly found, the LSD1 overexpressed in prostate cancer tissue, prostate cancer cells, body fluids of (or containing) prostate cancer tissue and/or cells or protein extracts thereof is targeted by anti-LSD1 antibodies. As a result, the amount of LSD1 expressed in accordance with the severity of the carcinoma and targeted by the respective antibodies can be detected directly or indirectly in accordance with methods of detecting protein-antibody complexes usually applied in the art. In this connection, the term "directly" has the meaning a skilled person would attribute to the term usually, i. e. any method by which a direct detection of such a complex is possible. (Non-restricting) examples of such direct methods are methods by which the presence of a certain molecule (complex) in a substance mixture can be established, as, for example, spectrometric (optical spectometry, mass spectrometry, etc.) methods or chromatographic (HPLC, column chromatography, thin layer chromatography etc.) methods or combinations thereof. In contrast, the term "indirectly" means, in accordance with the usual definition of a skilled person, any method by which an indirect detection of such a complex is possible. (Non-restricting) examples of such indirect methods are methods by which another molecule or chemical entity, including an antibody, is added to the complex mixture upon which step the additional molecule, entity (including the antibody) binds to the complex, which binding results into a (qualitatively and quantitatively) sensitive detection of the presence of the LSD1-anti-LSD1 antibody complex. Exemplary methods comprise (but are not restricted to) direct or indirect immunohistochemistry, immunocytochemistry and ELISA technologies or combinations thereof.

As mentioned above, the method may be applied to all parts of the mammalian body where the LSD1 overexpression occurs and/or overexpressed LSD1 can be detected. Examples are prostate (cancer) tissues, prostate (cancer) cells, body fluids from such tissues or cells wherein LSD1 can be found as well as protein extracts thereof. Prostate cancer tissue is preferred in accordance with the invention at present, without that the invention is restricted to prostate cancer tissue. The tissue or cell or body fluid sample may be obtained from biopsy samples from mammalian (most preferably human) prostate cancer specimens or from surgical specimens of prostatectomies. The sample may be a tissue section or may be cells or cell combinations, while the body fluid may be a fluid obtained from prostate cancer tissue (of any origin) or from corresponding cells, for example blood or serum. In addition, the method may be applied to protein extracts obtained from such tissues and/or cells such extracts being prepared by means of using solvents or solvent mixtures compatible with the biological/medical object and with the detection method described herein.

In accordance with the method of the invention, at least one anti-LSD1 antibody is employed in the method. There may be employed one anti-LSD1 antibody or there may be employed several anti-LSD1 antibodies. In preferred embodiments of the invention, the method employs one anti-LSD1 antibody. Any anti-LSD1 antibody known to a skilled person may be employed in accordance with the method of the present invention, which antibody may be a monoclonal antibody or a polyclonal antibody. Antibodies of any known origin may be used, for example rabbit antibodies, mouse antibodies, to name only two examples. However, in preferred embodiments, the at least one anti-LSD1 antibody or the one anti-LSD1 antibody is selected from the group consisting of polyclonal or monoclonal antibodies matching with a certain epitope in the human LSD1 protein, particularly preferable the AGPGTAGGSENGSEVAAQPAGLSGPAE VGPGAVGERTPRKKEPPRASPPGGLAEPPGSAGPQAGPTVVPGSATPMETG IAETPEGRRTSRRKRAKVEYREMDESLANLSEDEYYSE epitope in the human LSD1 protein. In particularly preferred embodiments of the invention, the anti-LSD1 antibody used is selected from the following antibodies which are all matching with the above epitope of the human LSD1 protein: rabbit polyclonal anti-LSD1 antibody 5996, rabbit polyclonal anti-LSD1 antibody 5996, and mouse polyclonal anti-LSD1 antibody 5994. The rabbit polyclonal anti-LSD1 antibody 5996 is particularly preferred. The anti-LSD1 antibodies 5996, 5995 and 5994 are given by way of examples only, but the invention is not restricted to those antibodies.

In the method of the invention, the at least one anti-LSD1 antibody is applied to the tissue, cell(s), body fluid and/or protein extracts thereof. This can preferably be performed by steps known per se to a person having ordinary skill in this field. Once the antibody is combined with the respective other component(s) (i. e. the tissue, cell(s), body fluid(s) and/or protein extract(s) thereof), the detection step is performed, wherein either a direct or an indirect detection is selected from the methods described above. In one method presently preferred, another antibody is added to the LSD1-anti-LSD1 antibody complex which, again is specific enough, more preferably which is highly specific for the detection of the complex. By such a method, a highly specific detection of the LSD1 present in the tissue, cell(s), body fluid(s) and/or protein extracts thereof can be obtained. Said highly specific detection is the basis for the use of this method in the surprisingly specific discrimination of progressive or less progressive prostate carcinomas.

As an even more preferred embodiment, a typical immunohistochemical analysis can be performed, by a person having ordinary skill in this technical field²⁹, on tissue specimens or cells collected from prostate cancer tissues according to the following protocol: 5 µm tissue sections were cut from formalin-fixed paraffin-embedded specimens and treated in a microwave oven (6 x 4 min, 750 W, in 10 mM citrate buffer) for antigen retrieval. After incubation with the primary anti-LSD1 antibodies described above (1 : 500 overnight at 4 °C) the specific immunoreaction was detected using a secondary anti-rabbit IgG (1 : 500, commercially available from Dako, Copenhagen, Denmark) and visualized with the ABC complex (commercially available from Vector Laboratories Burlingame, Vermont) diluted in 1 : 50 phosphate buffered saline (PBS).

In another preferred embodiment of the invention, a direct detection of the LDS1-anti-LSD1-antibody with suitable means, more preferably spectroscopic means (e. g. (not restricting) mass spectrometry) or with chromatographic means (e. g. (not restricting) HPLC or thin layer chromatography) may be conducted. Also combinations of such procedures are suitable, as a skilled person will recognize.

In another aspect, the invention also relates to the use of at least one antibody for identifying and/or scoring prostate carcinomas in a mammal.

In accordance with the invention, at least one anti-LSD1 antibody is used. There may be used one anti-LSD1 antibody or there may be used several anti-LSD1 antibodies. In preferred embodiments of the invention, the use comprises one anti-LSD1 antibody. Any anti-LSD1 antibody known to a skilled person may be employed in accordance with the present invention, which may be a monoclonal antibody or a polyclonal antibody. Antibodies of any known origin may be used, for example rabbit antibodies, mouse antibodies, to name only two examples. However, in preferred embodiments, the at least one anti-LSD1 antibody or the one anti-LSD1 antibody is selected from the group consisting of polyclonal or monoclonal antibodies matching with a certain epitope in the human LSD1 protein, particularly preferable the AGPGTAGGSENGSEVAAQPAGLSGPAE VGPGAVGERTPRKKEPPRASPPGGLAEPPGSAGPQAGPTVVPGSATPMETG IAETPEGRRTSRRKRAKVEYREMDESLANLSEDEYYSE epitope in the human LSD1 protein. In particularly preferred embodiments of the invention, the anti-LSD1 antibody used is selected from the following antibodies which are all matching with the above epitope of the human LSD1 protein: rabbit polyclonal anti-LSD1 antibody 5996, rabbit polyclonal anti-LSD1 antibody 5996, and mouse polyclonal anti-LSD1 antibody 5994. The rabbit polyclonal anti-LSD1 antibody 5996 is particularly preferred. The anti-LSD1 antibodies 5996, 5995 and 5994 are given by way of examples only, but the invention is not restricted to those antibodies.

Areas for a diagnostic use of the antibodies of the present invention are in assays as for example Western blotting, immunohistochemistry, immunocytochemistry, enzyme-linked immunosorbent assay (ELISA), immunoprecipitation, Chromatin Immunoprecipitation (ChIP), screening of libraries, and flow cytometric analyses.

The invention is described in more detail below, without restricting it to those embodiments specifically addressed in the above description as well as in the subsequent description of preferred embodiments.

To examine the expression pattern of LSD1, there were performed Northern blot analyses. *LSD1* mRNA is ubiquitously expressed in human and murine fetal and adult tissues (Fig. 2a and data not shown) as a transcript of 3.3 kb (Fig. 6a). To investigate LSD1 localisation in prostate and prostate tumours, there were used immunohistochemical analyses of 100 prostate cancer biopsies on tissue microarrays. As shown in figure 2b, LSD1 is detected in the epithelium of normal prostate and in tumour cells. Importantly, these cells also express AR (Fig. 2b) indicating that LSD1 and AR co-localise.

Next, the sub-cellular localisation of endogenous LSD1 and AR in human LNCaP prostate cancer cells was studied by immunofluorescence (Fig. 6b). LSD1 is present in the nucleus in the absence and presence of the AR agonist R1881. Addition of R1881 results in nuclear co-localisation of AR and LSD1. Taken together, the data show that LSD1 is a nuclear protein that co-localises with AR in androgen-sensitive tissues such as prostate.

Since LSD1 was found to associate with chromatin and demethylates H3-K4 *in vitro⁷,* it was examined whether LSD1 directly interacts with core histones. Interaction analyses demonstrated physical association with core histones *in vitro* (Fig. 7a). Furthermore, the analyses show that LSD1 interacts with the N-terminal tail of histone H3 (Fig. 7b).

To determine whether LSD1 and AR associate with chromatin *in vivo,* LNCaP cells treated with or without (i. e. untreated) R1881 were subjected to chromatin immunoprecipitation (ChIP). As shown in figure 3a, genomic DNA corresponding to the androgen response elements (ARE I+II and ARE III) located in the promoter and enhancer of the prostate specific antigen (PSA) gene, respectively, was immunoprecipitated in a ligand-dependent manner with α-AR antibodies. Genomic DNA derived from a region between the enhancer and promoter was not enriched (Fig. 3a). Association of LSD1 with the chromatinized PSA promoter is specific, since DNA from neither exon 4 of the PSA gene nor the promoters of the GAPDH and U6 genes is enriched (Fig. 3a).

To demonstrate that LSD1 and AR form ligand-dependent complexes on chromatinized AREs, agonist-treated LNCaP cells were subjected to sequential chromatin immunoprecipitation (Re-ChIP), first with an α-AR antibody and next with either α-LSD1 antibody or control α-rabbit IgG. Importantly, both ARE containing regions were enriched, demonstrating that LSD1 and AR form a ligand-dependent complex on chromatin (Fig. 3a).

Since PSA gene expression is induced by AR, the methylation levels of repressive histone marks were analysed, such as histone 3 at lysine 9 (H3-K9), histone 3 at lysine 27 (H3-K27), and histone 4 at lysine 20 (H4-K20). Stimulation of LNCaP cella with R1881 results in androgen-induced transcription and is accompanied by a robust decrease in mono-, di-, and trimethyl H3-K9 at the PSA promoter (Fig. 3b). In addition, there was observed a ligand-dependent decrease in dimethyl H4-K20, whereas mono- and trimethyl H4-K20 and methylation levels of H3-K27 remain unchanged (Fig. 8).

Since LSD1 is an AO that catalyses demethylation, a test was conducted whether monoamine oxidase inhibitors such as pargyline (N-methyl-N-2-propynylbenzylamine), clorgyline (N-methyl-N-propargyl-3-(2,4-dichlorophenoxy-)propylamine) or deprenyl (= seregeline; (R)-(-)-N,2-dimethyl-N-2-propynylphenethylamine) might interfere with LSD1 demethylation function. Importantly, pargyline blocks demethylation of mono- and dimethyl H3-K9 during androgen-induced transcription, whereas methylation levels of trimethyl H3-K9 and the methylation status of H3-K27 and, H4-K20 remain unchanged (Fig. 3b and Fig. 8). Interestingly, methylation of histone H3 at lysine 4 (H3-K4) is not altered in the presence of R1881 and not influenced by pargyline *in vivo* (Fig. 3b).

To prove that LSD1 executed the ligand-dependent demethylation of mono- and dimethyl H3-K9, we designed various siRNAs directed against LSD1 or an unrelated control (Fig. 9). Transfection of LNCaP cells leads to efficient and specific down-regulation of endogenous LSD1 but does not affect the level of endogenous AR (Fig. 3c). LSD1 knockdown blocks ligand-dependent demethylation of mono- and dimethyl H3-K9, but not that of trimethyl H3-K9 (Fig. 3c). The amount of total H3 on the PSA promoter is not influenced by the LSD1 knockdown (Fig. 3c).

To further validate that the LSD1/AR complex removes H3-K9 dimethyl marks in the presence of R1881, there was established a demethylation assay *in vitro.* Tandem affinity purified (TAP) LSD1 in the presence or absence of AR (Fig. 3d) was incubated in the presence of R1881 with HeLa nucleosomes as the substrate. The TAP-LSD1/AR complex demethylated dimethyl H3-K9 *in vitro,* whereas TAP-LSD1 or the TAP control failed to do so. The methylation status of the trimethyl H3-K9 control is not altered (Fig. 3d). An addition of pargyline blocked the demethylation of dimethyl H3-K9 by the TAP-tagged LSD1/AR complex (Fig. 3d). Thus, the *in vitro* assay proofs that the LSD1/AR complex directly and specifically demethylates H3-K9 and that the demethylation is blocked by pargyline.

Taken together, these data show the ligand-dependent association of LSD1 and AR on chromatinized AREs at the promoter of the PSA gene and the specific demethylation of the repressive histone marks mono- and dimethyl H3-K9.

Next, there were performed transient transfection assays to test whether LSD1 modulates the transcriptional activity of AR. Co-expression of LSD1 and AR results in a strong ligand-dependent activation of an MMTV-luciferase reporter (Fig. 4a), which is not observed with deletion mutant LSD1ΔAO or in the absence of either ligand or AR (Figure 4a and Fig. 10). Stimulation of AR activity by LSD1 is potent in different cell lines, and both AR-responsive minimal, synthetic and complex promoters were activated by LSD1 in a ligand-dependent manner (Fig. 10b, 10c). LSD1 does not affect the transcriptional activity of the related steroid hormone receptors, indicating that stimulation of AR is selective (Fig. 10h).

Furthermore, it is demonstrated that the AO domain (LSD1 247-852) of LSD1 suffices to stimulate AR- and ligand-dependent reporter gene activity (Fig. 4b).

Since displacement of repressive histone marks by LSD1 increases AR-dependent gene expression, inhibition of LSD1 should reduce AR activity. Consequently, monoamine oxidase inhibitors such as pargyline, clorgyline, and deprenyl severely impair LSD1-induced activation of AR (Fig. 4c). Importantly, in LNCaP cells, which express endogenous AR, only androgen-dependent but not unrelated reporters such as TK-LUC are inhibited by pargyline thus demonstrating specificity (Fig. 10j). Pargyline does not influence activity of other nuclear receptors (Fig. 10k). Moreover, qRT-PCR analyses demonstrate that pargyline also blocks the androgen-induced expression of the endogenous PSA gene in LNCaP cells (Fig. 4d).

Next, endogenously expressed LSD1 was efficiently used in LNCaP cells by vector (pSUPER-LSD1) mediated RNAi (Fig. 4e). Paralleling LSD1 knockdown, a significant ligand-dependent decrease of PSA-LUC reporter gene expression was observed (Fig. 4e), whereas expression of the unrelated TK-LUC is not influenced (data not shown). To address whether LSD1 governs androgen-dependent cell growth, LNCaP cells were infected with a lentivirus (pLV-THM-LSD1) expressing siRNA directed against LSD1. Infection with pLV-THM-LSD1 causes efficient and specific down-regulation of endogenous LSD1 but does not affect the level of endogenous AR (Fig. 4). Importantly, when compared to cells transduced with the pLV-THM-control virus, androgen-induced proliferation of LNCaP cells is dramatically inhibited by pLV-THM-LSD1 mediated LSD1 knockdown (Fig. 4f). These results show the physiological importance of LSD1 in the control of androgen-induced gene regulation and cell proliferation.

In summary, the above data demonstrate that AR function is controlled by the demethylase LSD1. LSD1 and AR associate at chromatinized AREs of AR target genes a ligand-dependent manner, which results in concomitant demethylation of the repressive histone marks mono- and dimethyl H3-K9. LSD1 has been described as a component of co-repressor complexes⁸⁻¹¹ and a recent model proposes that LSD1 represses transcription of genes silenced by Co-REST due to demethylation of the activating histone marks on H3-K4⁷. However, when complexed with AR, LSD1 demethylates the repressing histone marks mono- and dimethyl H3-K9 and thereby promotes gene activation. Thus, depending on the specific interacting partners, LSD1 action might result in either gene silencing or activation. Of importance is the observation that inhibitors such as pargyline control the demethylase activity of LSD1 and thereby regulate AR. Thus, specific modulation of LSD1 activity might be a promising therapeutic target in tissues such as brain, testis, prostate where AR plays a pivotal physiological role.

### Methods

### Plasmids

The following plasmids were described previously: pSG5-AR, PR, CMX-Flag, GST-AR-NTD, GST-AR-DBD, GST-AR-LBD, GST-ERβ-NTD, MMTV-LUC, and TK-LUC¹⁷ ; ARE_{2X}-TATA-LUC, ARE_{2X}-TK-LUC²¹; Slp-ARU-TATA-LUC²²; PSA-LUC²³; pLV-THM (http://www.tronolab.unige.ch/); pSUPER²⁴; GST-Nix1, RARα, ERα, and TRβ²⁵; βRE-LUC, TREp-LUC, and ERE₂ₓ-TATA-LUC²⁶. To construct CMX-Flag-LSD1, CMX-Flag-LSD1 1-174, CMX-Flag-LSD1 175-246, and CMX-Flag-LSD1 247-854, CMX-Flag-LSD1Δ281-360, CMX-Flag-LSD1ΔAO (LSD1 1-247) and GST-RORβ (RORβaa76-459), the corresponding fragments were amplified by PCR and inserted into CMX-Flag or pGEX4T-1. pSUPER-control, pSUPER-LSD1 and pLV-THM-LSD1 were constructed according to (hftp://www.tronolab.unige.ch/) and as published²⁴. To construct TAP-LSD1 and TAP-FHL2, the corresponding fragments were amplified by PCR and inserted into a modified pCMX expression plasmid containing an N-terminal TAP tag (TAP). Sequences can be obtained upon request.

### Immunofluorescence

Cells were analysed essentially as described¹³. Primary antibody staining was performed with the indicated dilutions: α-AR 441 (1:500) and α-LSD1 (1:500). Sub-cellular localisation was visualised using secondary Alexa Fluor 488- and 546-labelled antibodies (1:6000; Molecular Probes). Nuclei were stained with 1 µg/ml DAPI (Roche).

### In vitro pull-down assays

GST pull-down assays were performed with equal amounts of GST or GST fusion proteins as described¹⁷ using buffer containing either 150 mM KCI, 0.15% NP40 (Fig. 1 b) or 600 mM KCI, 0.3% NP40 (Fig. 7a). Pull-downs with sepharose coupled histone H3 tail were performed as described²⁷ in 20 mM Tris pH 8.5, 150 mM NaCl, 0.5% NP40. 10 % of the *in vitro* translated proteins were loaded as input.

### mRNA analyses

Northern blot analyses were performed with a Human Multiple Tissue Expression Array and a Human Multiple Tissue Northern Blot (BD Biosciences Clontech) with an LSD1-specific probe spanning either bp 1-741 or bp 1-2556, labelled with StripEZ (Ambion), and hybridized as recommended.

### Tandem Affinity Purification

TAP purification was essentially performed as described²⁸. 293 cells transfected with either TAP-tag-FHL2 (TAP-FHL2) or control TAP-tag (TAP) were lysed in buffer A (20 mM HEPES/KOH pH 7.9, 420 mM NaCl, 1.5 mM MgCl₂, 10 mM KCI, 25% Glycerol, 0.5 mM DTT, 0.1 % NP-40, 0.1 mM EDTA, 50 mM NaF, 0.2 mM Na₃VO₄, and Complete^{®} protease inhibitor cocktail). TAP-tagged proteins were bound to IgG-sepharose (GE-Healthcare) in salt-adjusted buffer A (150 mM NaCl) at 4°C over night followed by repeated washing. Bound complexes were relieved from IgG-sepharose by TEV-protease (100 U, Invitrogen) in TEV buffer (10 mM TrisHCl pH 8.0, 150 mM NaCl, 0.1% NP40, 1 mM DTT, 1 mM EDTA). CBP-containing complexes were immobilized on a Calmodulin affinity resin (Stratagene) for 4h at 16°C. Complexes were washed five times with CBP-buffer (10 mM TrisHCl pH 8.0, 150 mM NaCl, 0.1% NP40, 1 mM MgAcetate, 1 mM Imidazole, 10 mM β-mercaptoethanol, 2 mM CaCl₂) and eluted with CBP buffer containing 10 mM EGTA instead of CaCl₂). Protein complexes were size separated by SDS-PAGE, visualized by Coomassie stain and identified by MALDI-TOF/TOF (Explora AG, Darmstadt, Germany).

### Immunohistochemistry

Polyclonal rabbit□-α-LSD1 antibody was generated according to standard procedures. Stainings were performed using a protocol¹³ for antigen retrieval and indirect immunoperoxidase. α-AR 441 (Santa Cruz) and α-LSD1 were used at a dilution of 1:75 and 1:500, rabbit IgG and mouse IgG (1:500; Dako) were used as secondary antibodies and immunoreactions were visualised with the ABC-complex diluted 1:50 in PBS (Vectastain, Vector).

### Cell culture and transfections

293 and CV-1 cells were cultured and transfected as described¹². LNCaP cells were cultured in phenol-red-free RPMI1640 supplemented with 10% double-stripped fetal calf serum (dsFCS) and transfected with Effectene (Qiagen). The following amounts per well were used: MMTV-LUC, ARE₂ₓ-TATA-LUC, ARE₂ₓ-TK-LUC, TK-LUC, TREp-LUC, β-RE-LUC, ERE₂ₓ-TATA-LUC, PSA-LUC, Slp-ARU-TATA-LUC 500 ng each, 25 ng expression plasmids for AR, PR, ERα, RARα, and TRβ; 500 to 700 ng expression plasmids for LSD1 1-174, LSD1 175-246, LSD1 247-852, LSD1Δ281-360, LSD1ΔAO, pSUPER-control, and pSUPER-LSD1; 100 to 700 ng expression plasmids for LSD1 were transfected per well. Chemicals were obtained as indicated: pargyline (Sigma); deprenyl and clorgyline (ICN Biomedicals Inc.); R1881, T3, E₂, all-trans RA and R5020 (Schering AG, Berlin). Cells were treated with or without 10⁻¹⁰ M R1881, 10⁻⁸ M R5020, 10⁻⁹ M E₂, 10⁻⁷ M T3, 10⁻⁶ M all-trans RA, 3 x 10⁻³ M pargyline, 1 x 10⁻³ M deprenyl, or 1 x 10⁻⁴ M clorgyline for 18 hours as indicated. Luciferase activity was assayed as described⁹. All experiments were repeated at least five times in duplicate.

### Chromatin Immunoprecipitation

ChIP experiments were performed essentially as described¹⁴ . LNCaP cells were treated for 18 hours with or without pargyline and for 210 min with or without 10⁻⁸ M R1881 as indicated. LNCaP cells were transfected three days before harvesting for CHIP with or without siRNA (Qiagen) following the manufacture's instructions. Immunoprecipitation was performed with specific antibodies (α-monoMeK9H3, α-diMeK9H3, α-triMeK9H3, α-monoMeK4H3, α-diMeK4H3, α-triMeK4H3, α-H3 (abcam), α-LSD1, and α-AR PG21 (Upstate Biotechnology) on GammaBind^{™}-Sepharose 4B (GE-Healthcare). For PCR, 1-5 µl out of 50 µl DNA extract was used. For Re-ChIP assays, immunoprecipitations were sequentially washed with TSE I, TSE II, buffer III, and TE¹⁴. Complexes were eluted by incubation with 10 mM DTT at 37°C for 30 min, diluted 50 times with dilution buffer¹⁴ followed by a second immunoprecipitation with the indicated antibody. Primer sequences were as follows: exon 4, *PSA* (+3909/+4067) 5'-GTGTGTGGACCTCCATGTTATT-3' and 5'-CCACTCACCTTTCCCCTCAAG-3'; middle, *PSA* (-2223/-1951) 5'-TGGGTTGGGTCAGGTTTTGGTT-3' and 5'-TCTTCCCCTGTTTCTAGTTGAGTG-3'; PCR primers for ARE I+II (*PSA* (-459/-121)) and ARE III (*PSA* (-4288/-3922)), *GAPDH,* and U6 have been previously described ^{7, 15, 16}.

### Co-immunoprecipitation assays and Western blot analyses

Experiments were performed essentially as described¹⁷. Immunoprecipitations from extracts of murine testis were performed in the presence of 1 x 10⁻⁹ M R1881 with either α-LSD1, α-cyclin A¹⁷ antibodies, or rabbit IgG. Western blots were decorated as indicated. α-AR (N20, Santa Cruz) was used. 10 % of testis extract was loaded as input.

### Cell proliferation assay

pLV-THM-control and pLV-THM-LSD1 were used to produce recombinant lentiviruses to infect LNCaP cells as described¹⁸. The infected cells were cultured for 72 hours in medium supplemented with 10% dsFCS. 0.3 x 10⁴ cells were plated in a 96-well plate with or without 10⁻⁷ M R1881. The cell proliferation Elisa BrdU Colorimetric Assay (Roche) was performed according to the manufacturer's instructions. The experiments were repeated three times in quadruplet.

### qRT-PCR and statistical analysis

DNAsel-treated RNA isolated using RNAwiz (Ambion) was used for reverse transcription. Quantitative PCR was performed in an ABI PRISM 7700 sequence detector. Product formation was detected by incorporation of SYBR Green I using ROX as a passive reference (ABgene). The expression ratios of the analyzed cDNAs were related to the normalized Cₚ of the housekeeping gene *GAPDH* in control and sample. The following primers were used: *GAPDH:* 5'-GAAGGTGAAGGTCGGACTC-3'; 5'-GAAGATGGTGATGGGATTTC-3'; *PSA:* 5'-CACCTGCTCGGGTGATTCTG-3'; 5'-CCACTTCCGGTAATGCACCA-3'. Statistical analysis for qPCR was performed by group-wise comparison based on PCR efficiencies and the mean crossing point deviation between sample and control group using Relative Expression Software Tool¹⁹. Experiments were repeated and analysed three times.

### Demethylase assay

The demethlylation assay was essentially performed as described⁷. TAP-tagged proteins were bound to IgG-sepharose, washed and incubated in buffer 1 supplemented with 10 mM ATP, 10⁻⁹ M R1881 with or without 1 x 10⁻³ M pargyline and 1 µg of nucleosomes purified from HeLa cells¹⁶ for 6 hours at 37°C. The reaction mixture was analyzed by SDS-PAGE followed by Western blotting using antibodies as indicated.

### References

1. Breslow, M., Chan, C.W., Dhom, G. et al. Latent carcinoma of prostate at autopsy in seven areas. Int. J. Cancer. 1977, 20:680-688
2. Cato, A.C. and Peterzierl, H. The androgen receptor as a mediator of gene expression and signal transduction pathways. Trends Endocrinol. Metab. 1998, 9, 150-154.
3. Denmeade. S.R. and and Isaacs, J.T. A history of prostate cancer treatment. Nat. Rev. Cancer 2002, 5, 389-96
4. Waterbor, J.W. and Bueschen, A.J. Prostate Cancer Screening (United States). Cancer Causes Control 1995, 6:267-274
5. Glass, C.K. & Rosenfeld, M.G. The coregulator exchange in transcriptional function of nuclear receptors. Genes Dev. 14, 121-141 (2000).
6. Strahl, B.D. & Allis, C.D. The language of covalent histone modifications. Nature 403, 41-45 (2000).
7. Shi, Y. et al. Histone demethylation mediated by the nuclear amine oxidase homolog LSD1. Cell 119, 941-953 (2004).
8. Shi, Y. et al. Coordinated histone modifications mediated by a CtBP corepressor complex. Nature 422, 735-738 (2003).
9. Hakimi, M.A. et al. A candidate X-linked mental retardation gene is a component of a new family of histone deacetylase-containing complexes. J. Biol. Chem. 278, 7234-7239 (2003).
10. Hakimi, M.A. et al. A core-BRAF35 complex containing histone deacetylase mediates repression of neuronal-specific genes. Proc. Natl Acad. Sci. U S A 99, 7420-7425 (2002).
11. Eimer, S. et al. Loss of spr-5 bypasses the requirement for the C.elegans presenilin sel-12 by derepressing hop-1. EMBO J. 21, 5787-5796 (2002).
12. Müller, J.M. et al. FHL2, a novel tissue-specific coactivator of the androgen receptor. EMBO J. 19, 359-369 (2000).
13. Müller, J.M. et al. The transcriptional coactivator FHL2 transmits Rho signals from the cell membrane into the nucleus. EMBO J. 21, 736-748 (2002).
14.Shang, Y., Myers, M. & Brown, M. Formation of the androgen receptor transcription complex. Mol. Cell. 9, 601-610 (2002)
15. Shatkina, L. et al. The cochaperone Bag-1 L enhances androgen receptor action via interaction with the NH2-terminal region of the receptor. Mol. Cell. Biol. 23, 7189-7197 (2003).
16. Kang, Z., Pirskanen, A., Jänne, O.A. & Palvimo, J.J. Involvement of proteasome in the dynamic assembly of the androgen receptor transcription complex. J. Biol. Chem. 277, 48366-48371 (2002).
17. Metzger, E. et al. A novel inducible transactivation domain in the androgen receptor: implications for PRK in prostate cancer. EMBO J. 22, 270-280 (2003).
18. Wiznerowicz, M. & Trono, D. Conditional suppression of cellular genes: lentivirus vector-mediated drug-inducible RNA interference. J. Virol. 77, 8957-8961 (2003).
19. Pfaffl, M.W., Horgan, G.W. & Dempfle, L. Relative expression software tool (REST) for group-wise comparison and statistical analysis of relative expression results in real-time PCR. Nucleic Acids Res. 30, e36 (2002).
20.O'Neill, T.E., Roberge, M. & Bradbury E.M. Nucleosome arrays inhibit both initiation and elongation of transcripts by bacteriophage T7 RNA polymerase. J. Mol. Biol. 223, 67-78 (1992).
21. Schüle, R. et al. Functional antagonism between oncoprotein c-Jun and the glucocorticoid receptor. Cell 62, 1217-1226 (1990).
22.Verrijdt, G. et al. Functional interplay between two response elements with distinct binding characteristics dictates androgen specificity of the mouse sex-limited protein enhancer. J. Biol. Chem. 277, 35191-35201 (2002).
23. Sun, Z., Pan, J. & Balk, S.P. Androgen receptor-associated protein complex binds upstream of the androgen-responsive elements in the promoters of human prostate-specific antigen and kallikrein 2 genes. Nucleic Acids Res. 25, 3318-3325 (1997).
24. Brummelkamp, T.R., Bernards, R. & Agami, R. A system for stable expression of short interfering RNAs in mammalian cells. Science 296, 550-553 (2002).
25. Greiner, E.F. et al. Differential ligand-dependent protein-protein interaction between nuclear receptors and a neuronal-specific cofactor. Proc. Natl Acad. Sci. U S A 97, 7160-7165 (2000).
26. Schüle, R. et al. Jun-Fos and receptors for vitamin A and D recognize a common response element in the human osteocalcin gene. Cell 61, 497-504 (1990).
27.Schneider, R. et al. Direct binding of INHAT to H3 tails disrupted by modifications. J. Biol. Chem. 279, 23859-23862 (2004).
28. Rigaut, G. et al. A generic protein purification method for protein complex characterization and proteome exploration. Nat. Biotechnol. 17, 1030-1032 (1999).
29. Bocker, T., Bittinger, A., Wieland, W., Buettner, R., Fauser, G., Hofstaedter, F., Rüschoff, In vitro and ex vivo expression of nucleolar proteins B23 and p120 in benign and malignant epithelial lesions of the prostate. J. Modern Pathology 8: 226-31, 1995.

## Claims

1. A method for identifying and/or scoring prostate carcinomas, said method comprising the step of immunostaining tissues, cells, body fluids and/or protein extracts relating to prostate cancer including employing anti-LSD1 antibodies, and quantifying the LSD1 amount in said tissues, cells, body fluids and/or protein extracts, wherein said method is carried out outside the human or animal body.

2. The method according to claim 1, wherein the step of quantifying is a direct or indirect quantifying.

3. The method of claim 1 or claim 2, wherein the direct quantifying comprises methods by which the presence of a certain molecule or molecule complex in a substance mixture can be established, preferably spectrometric (optical spectometry, mass spectrometry) methods or chromatographic (HPLC, column chromatography, thin layer chromatography) methods or combinations thereof.

4. The method of claim 1 or claim 2, wherein the indirect quantifying comprises methods by which another molecule or chemical entity, preferably anantibody, is added to the complex mixture upon which step the additional molecule or entity (optionally including the antibody) binds to the complex, resulting into a (qualitatively and quantitatively) sensitive detection of the presence of the LSD1-anti-LSD1 antibody complex, preferably comprises immunohistochemistry, immunocytochemistry and ELISA technologies or combinations thereof.

5. The method according to any of the claims 1 to 4, wherein the step of immunostaining tissues, cells, body fluids and/or protein extracts relating to prostate cancer comprises immunostaining prostate cancer tissue(s), prostate cancer cell(s), prostate cancer tissue and/or cell body fluid(s) and/or protein extracts from prostate cancer tissue(s), cell(s) and/or body fluids.

6. Use of at least one anti-LSD1 antibody capable of targeting an epitope in the LSD 1 protein of a mammal and/or of a pharmaceutical or diagnostic composition comprising at least one anti-LSD1 antibodycapable of targeting an epitope in the LSD1 protein of a mammal, optionally together with at least one of pharmaceutically or diagnostically acceptable carriers, diluents and/or auxiliary substances, for identifying and/or scoring prostate carcinomas in a mammal, wherein said identifying and/or scoring is carried out outside the human or animal body.

7. The use of claim 6, wherein the mammal is a human.

## Patentansprüche

1. Verfahren zur Identifizierung und/oder Klassifizierung von Prostatakarzinomen, wobei das genannte Verfahren den Schritt der Immunfärbung der mit Prostatakrebs in Zusammenhang stehenden Gewebe, Zellen, Körperflüssigkeiten und/oder Proteinextrakte unter Verwendung von Anti-LSD1-Antikörpern und den Schritt der Quantifizierung der LSD 1 Menge in den genannten Geweben, Zellen, Körperflüssigkeiten und/oder Proteinextrakten umfasst, wobei das genannte Verfahren außerhalb des menschlichen oder tierischen Körpers durchgeführt wird.

2. Verfahren nach Anspruch 1, wobei der Schritt der Quantifizierung eine direkte oder indirekte Quantifizierung ist.

3. Verfahren nach Anspruch 1 oder Anspruch 2, wobei die direkte Quantifizierung Verfahren umfasst, mit denen das Vorhandensein eines bestimmten Moleküls oder Molekülkomplexes in einem Stoffgemisch ermittelt werden kann, vorzugsweise spektrometrische (optische Spektrometrie, Massenspektrometrie) Verfahren oder chromatographische (HPLC, Säulenchromatographie, Dünnschichtchromatographie) Verfahren oder Kombinationen davon.

4. Verfahren nach Anspruch 1 oder Anspruch 2, wobei die indirekte Quantifizierung Verfahren umfasst, bei denen ein anderes Molekül oder ein anderer chemischer Stoff, vorzugsweise ein Antikörper, dem Komplexgemisch zugesetzt wird, worauf folgend das zusätzliche Molekül oder der zusätzliche Stoff (gegebenenfalls einschließlich des Antikörpers) an den Komplex bindet, was zu einer (qualitativ und quantitativ) empfindlichen Detektion der Anwesenheit des LSD1-Anti-LSD1-Antikörper-Komplexes führt, vorzugsweise umfassend Immunhistochemie, Immunzytochemie und ELISA-Technologien oder Kombinationen davon.

5. Verfahren nach einem der Ansprüche 1 bis 4, wobei der Schritt der Immunfärbung der mit Prostatakrebs in Zusammenhang stehenden Gewebe, Zellen, Körperflüssigkeiten und/oder Proteinextrakte die Immunfärbung von Prostatakrebsgewebe(n), Prostatakrebszelle(n), Prostatakrebsgewebe und/oder -zellkörperflüssigkeit(en) und/oder Proteinextrakten von Prostatakrebsgewebe(n), -zelle(n) und/oder -körperflüssigkeiten umfasst.

6. Verwendung von mindestens einem Anti-LSD1-Antikörper, welcher fähig ist an ein Epitop im LSD1-Protein eines Säugers zu binden, und/oder von einer pharmazeutischen oder diagnostischen Zusammensetzung, die mindestens einen Anti-LSD1-Antikörper, welcher fähig ist an ein Epitop im LSD1-Protein eines Säugers zu binden, umfasst, gegebenenfalls zusammen mit mindestens einem pharmazeutisch oder diagnostisch verträglichen Träger, Verdünnungsmittel und/oder Hilfsstoff, zur Identifizierung und/oder Klassifizierung von Prostatakarzinomen in einem Säuger, wobei die genannte Identifizierung und/oder Klassifizierung außerhalb des menschlichen oder tierischen Körpers durchgeführt wird.

7. Verwendung nach Anspruch 6, wobei der Säuger ein Mensch ist.

## Revendications

1. Procédé pour identifier et/ou évaluer des carcinomes de la prostate, ledit procédé comprenant l'étape d'immunocoloration de tissus, cellules, fluides corporels et/ou extraits protéiques associés au cancer de la prostate, qui consiste à employer des anticorps anti-LSD1 et à quantifier la quantité de LSD1 dans lesdits tissus, cellules, fluides corporels et/ou extraits protéiques, ledit procédé étant réalisé en dehors du corps humain ou animal.

2. Procédé selon la revendication 1, dans lequel l'étape de quantification est une quantification directe ou indirecte.

3. Procédé selon la revendication 1 ou la revendication 2, dans lequel la quantification directe comprend des procédés par lesquels la présence d'une certaine molécule ou d'un complexe de molécules dans un mélange de substances peut être établie, de préférence des procédés spectrométriques (spectrométrie optique, spectrométrie de masse) ou des procédés chromatographiques (HPLC, chromatographie sur colonne, chromatographie sur couche mince) ou des combinaisons de ceux-ci.

4. Procédé selon la revendication 1 ou la revendication 2, dans lequel la quantification indirecte comprend des procédés par lesquels une autre molécule ou entité chimique, de préférence un anticorps, est ajoutée au mélange de complexes, étape pendant laquelle la molécule ou entité supplémentaire (comprenant facultativement l'anticorps) se lie au complexe, en ayant pour résultat une détection sensible (de manière qualitative et quantitative) de la présence du complexe LSD1-anticorps anti-LSD1, et comprend de préférence des technologies d'immunohistochimie, d'immunocytochimie et ELISA, ou des combinaisons de celles-ci.

5. Procédé selon l'une quelconque des revendications 1 à 4, dans lequel l'étape d'immunocoloration des tissus, cellules, fluides corporels et/ou extraits protéiques associés au cancer de la prostate, comprend une immunocoloration d'un/de tissu(s) du cancer de la prostate, d'une/de cellule(s) du cancer de la prostate, d'un/de fluide(s) corporel(s) tissulaire(s) et/ou cellulaire(s) du cancer de la prostate et/ou d'extraits protéiques de tissu(s), cellule(s) et/ou fluides corporels du cancer de la prostate.

6. Utilisation d'au moins un anticorps anti-LSD1 capable de cibler un épitope dans la protéine LSD1 d'un mammifère et/ou d'une composition pharmaceutique ou diagnostique comprenant au moins un anticorps anti-LSD1 capable de cibler un épitope dans la protéine LSD1 d'un mammifère, facultativement avec au moins un parmi des véhicules, diluants et/ou substances auxiliaires acceptables d'un point de vue pharmaceutique ou diagnostique, pour identifier et/ou évaluer des carcinomes de la prostate chez un mammifère, où ladite identification et/ou évaluation est réalisée en dehors du corps humain ou animal.

7. Utilisation selon la revendication 6, dans laquelle le mammifère est un humain.
